# EUROPEAN PATENT SPECIFICATION

(11) **EP 1 848 331 B1**
(45) Date of publication and mention of the grant of the patent: **08.04.2015**
(21) Application number: 06734485.3
(22) Date of filing: 06.02.2006
(51) Int. Cl.: A61B 17/70, A61B 5/00, A61B 5/05, A61B 17/00, A61N 1/36

(54) **APPARATUS FOR LOCATING DEFECTS IN BONE TISSUE**
VORRICHTUNG ZUM AUFFINDEN VON DEFEKTEN IN KNOCHENGEWEBE
APPAREIL DE LOCALISATION DES ANOMALIES DU TISSU OSSEUX

(30) Priority: 07.02.2005 US 52666
(43) Date of publication of application: 31.10.2007
(73) Proprietor: Warsaw Orthopedic, Inc., Warsaw, IN 46581 (US)
(72) Inventor: FELTON, Sharonda, T., Cordova, Tennessee 38016 (US); ADCOX, William, K., Memphis, Tennessee 38103 (US); NEUBARDT, Seth, L., White Plaines, New York 10605 (US)
(74) Representative: Hutchinson, Glenn Stanley
(86) International application number: PCT/US2006/004237
(87) International publication number: WO 2006/086363

(56) References cited:
- WO-A-03/037170
- DE-C1- 19 921 279
- US-A1- 2002 161 372

## Description

### BACKGROUND

Surgery for a patient can be painful and traumatic, particularly in the affected area of the patient's body. To accomplish spinal fixation, a necessary procedure often involves inserting spinal pedicle screws into a pedicle wall of a vertebra in a patient's spine. Pedicle screws are advantageous in that they are strong and provide stability, however, care must be taken to avoid nerve impingement during the placement of pedicle screws in the spine. Measures taken to locate any potential defects before insertion of a pedicle screw can facilitate screw insertion.

Locating defects such as openings in bone tissue that expose nerves can be difficult. Some procedures involve monitoring muscle reactions to electrical stimulation to locate nerves in an area of bone tissue. If a nerve is not located and a screw contacts an exposed nerve, the screw can impinge on the nerve or become too close to the nerve root causing pain and other implications for the patient. Additionally, it is often too late to reverse the nerve damage that is caused by removing the screw or conducting other remedial procedures. In a procedure in which a screw is inserted into a pedicle wall without first determining the proximity of neural elements, it is very difficult to determine the existence and/or proximity of any defects in the bone tissue, what areas of the pedicle wall have been breached or contain a defect, and where to redirect the pedicle screw to avoid the breached wall or defect. There remains a need for instruments and methods that can be employed for locating any defects in bone tissue surrounding a hole in which a screw or other anchoring mechanism will be inserted. The present invention is directed to meeting these needs, among others.

WO03/037170 describes a generally linear tool capable of performing pedicle integrity assessments.

### SUMMARY

The invention is defined in the appended claims.

According to the invention, an instrument to indicate defects within bone tissue is provided with a handle member and a probe member. The handle member has a rotatable coupling member for connection with the probe member. The probe member extends distally from the handle member and includes a shaft portion and an angled portion. The angled portion extends transversely from the shaft portion and has a probe end. The probe end carries an electrical signal and the handle member operates to rotate the probe member and the probe end within a hole in bone tissue to locate neural elements in the bone tissue.

Further, the invention involves a neural element and defect detection apparatus. The apparatus comprises a defect locating instrument with a handle member and a probe member. The probe member includes a longitudinal shaft portion and a distal angled portion. The distal angled portion includes a non-insulated probe end on its distal end that carries an electrical signal. The handle member rotates the probe member and the probe end about a longitudinal axis, while the probe end carries the electrical signal, to determine the proximity of neural elements and locate defects in bone tissue surrounding a hole. The apparatus further includes an operator display device to display a representation of the rotation of the probe end relative to the longitudinal axis and a representation of neural elements and defects in the bone tissue located by the probe end.

Further, the invention involves a system comprising a handle member, a probe member, and a nerve monitoring system. The handle member includes a circuit and a motor which rotates a coupling member within the handle member. The probe member has a proximal end and a distal end opposite the proximal end. The proximal end of the probe member is removably coupled to the coupling member of the handle member. Additionally, the probe member has a longitudinal shaft portion along a longitudinal axis at its proximal end and a distal angled portion extending transversely to the longitudinal axis adjacent its distal end. The distal angled portion includes a probe end at its distal end which carries an electrical signal and is designed to rotate about the longitudinal axis to locate neural elements and defects in an area of bone tissue surrounding a hole. The nerve monitoring system is electrically coupled to the probe member.

### BRIEF DESCRIPTION OF THE FIGURES

Fig. 1 is a schematic view of a defect locating system.
Fig. 2 is a partial, schematic view of a defect locating device.
Fig. 3 is a partial, schematic view of a defect locating device relative to a section of a spine.
Fig. 4 is a schematic view of a display.
Fig. 5 is a flowchart of one type of diagnostic procedure that can be implemented with the system of Fig. 1.
Fig. 6 is another embodiment probe member usable with a defect locating system.

### DESCRIPTION OF THE ILLUSTRATED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is hereby intended, such alterations and further modifications in the illustrated devices, and such further applications of the principles of the invention as illustrated herein being contemplated as would normally occur to one skilled hi the art to which tine invention relates.

An instrument for locating defects within bone tissue includes a handle member and a probe member. The handle member includes a rotatable coupling member connected with the probe member. The probe member extends distally from the handle member and includes a longitudinal shaft and an angled end. The angled end has a non-insulated probe end, such as a ball tip, on its distal end that is designed to rotate around a longitudinal axis. The probe end carries an electrical signal to determine the proximity of neural elements and rotates within a hole in bone tissue to locate defects hi the bone tissue. The detection of the occurrence and location of bone tissue defects in the hole directs the surgeon in forming the hole to receive a bone screw in a manner that avoids neural elements hi the bone tissue.

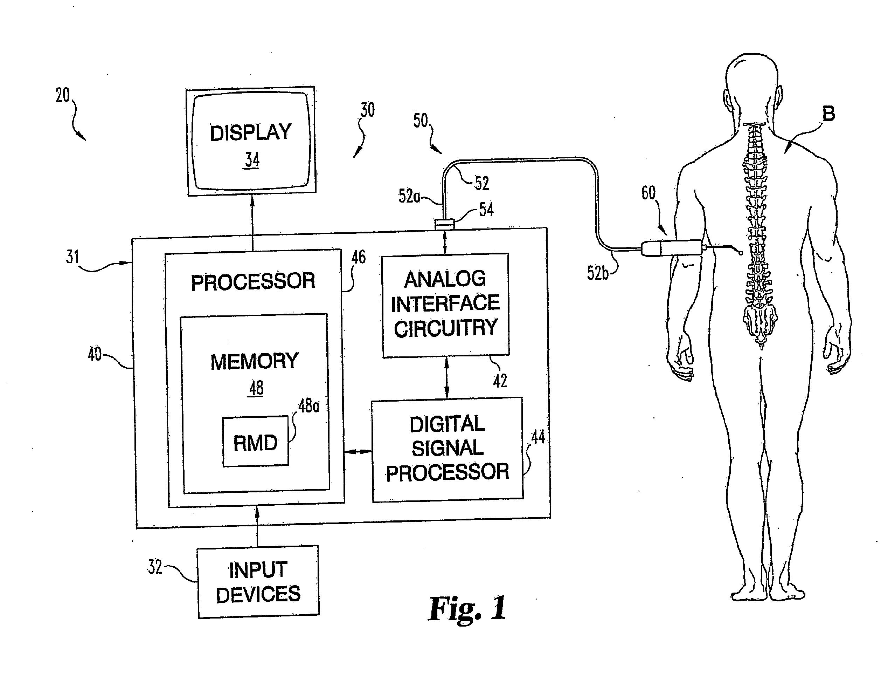

Fig. 1 illustrates a system 20 that includes an instrument, which is a defect locating device 60, and associated equipment arranged to provide medical treatment. System 20 is arranged to determine the proximity of neural elements and find defects within bone tissue surrounding a first hole in a pedicle wall of one or more vertebrae of spinal column B.

Based on the location of the defects found, system 20 is arranged to allow for redirection of a defect locating device to find an area of bone tissue without defects and thus a more suitable location for forming a hole in and placement of a screw in a pedicle or other bony structure. System 20 includes nerve monitoring system 30, connection link 50, and defect locating device 60. Nerve monitoring system 30 includes equipment 31 coupled to device 60 with connection link 50, or equipment 31 can be integrated with device 60. Device 60 is configured for placement within a hole in a spinal pedicle wall of spinal column B of a human patient or subject, as schematically represented in Fig. 1.

In one embodiment, system 30 is part of the NIM-Spine™ System marketed by Medtronic, Inc. Another example of a probe and nerve monitoring procedure is provided in U.S. Patent No. 5,474,558 to Neubardt.

Equipment 31 may include operator input devices 32, operator display device 34, and various other operator-utilized equipment of system 20 that is external to a patient during use. Input devices 32 may include an alphanumeric keyboard and mouse or other pointing device of a standard variety. Alternatively or additionally, one or more other input devices can be utilized, such as a voice input subsystem or a different type as would occur to those skilled in the art. Operator display device 34 can be of a Cathode Ray Tube (CRT) type, Liquid Crystal Display (LCD) type, plasma type, Organic Light Emitting Diode (OLED) type, or such different type as would occur to those skilled in the art. Alternatively or additionally, one or more other operator output devices can be utilized, such as a printer, one or more loudspeakers, headphones, or such different type as would occur to those skilled in the art. Nerve monitoring system 30 also can include one or more communication interfaces suitable for connection to a computer network, such as a Local Area Network (LAN), Municipal Area Network (MAN), and/or Wide Area Network (WAN) like the Internet; a medical diagnostic device; another therapeutic device; a medical imaging device; a Personal Digital Assistant (PDA) device; a digital still image or video camera; and/or audio device, to name only a few. Nerve monitoring system 30 can be arranged to show other information under control of the operator.

Equipment 31 may also include processing subsystem 40 for processing signals and data associated with system 20. Subsystem 40 may include analog interface circuitry 42, Digital Signal Processor (DSP) 44, data processor 46, and memory 48. Analog interface circuitry 42 can be responsive to control signals from DSP 44 to provide corresponding analog stimulus signals to device 60. At least one of analog interface circuitry 42 and DSP 44 may include one or more digital-to-analog converters (DAC) and one or more analog-to-digital converters (ADC) to facilitate operation of system 20 in the manner to be described in greater detail hereinafter. Processor 46 can be coupled to DSP 44 to bidirectionally communicate therewith, selectively provide output to display device 34, and selectively respond to input from operator input devices 32.

DSP 44 and/or processor 46 can be of a programmable type; a dedicated, hardwired state machine; or a combination of these. DSP 44 and processor 46 perform in accordance with operating logic that can be defined by software programming instructions, firmware, dedicated hardware, a combination of these, or in a different manner as would occur to those skilled in the art. For a programmable form of DSP 44 or processor 46, at least a portion of this operating logic can be defined by instructions stored in memory 48. Programming of DSP 44 and/or processor 46 can be of a standard, static type; an adaptive type provided by neural networking, expert-assisted learning, fuzzy logic, or the like; or a combination of these.

Memory 48 is illustrated in association with processor 46; however, memory 48 can be separate from or at least partially included in one or more of DSP 44 and processor 46. Memory 48 includes at least one Removable Memory Device (RMD) 48a. Memory 48 can be of a solid-state variety, electromagnetic variety, optical variety, or a combination of these forms. Furthermore, memory 48 can be volatile, nonvolatile, or a mixture of these types. Memory 48 can be at least partially integrated with circuitry 42, DSP 44, and/or processor 46. RMD 48a can be a floppy disc, cartridge, or tape form of removable electromagnetic recording media; an optical disc, such as a CD or DVD type; an electrically reprogrammable solid-state type of nonvolatile memory, and/or such different variety as would occur to those skilled in the art. In still other embodiments, RMD 48a is absent.

Circuitry 42, DSP 44, and processor 46 can be comprised of one or more components of any type suitable to operate as described herein. Further, it should be appreciated that all or any portion of circuitry 42, DSP 44, and processor 46 can be integrated together in a common device, and/or provided as multiple processing units. For a multiple processing unit form of DSP 44 or processor 46; distributed, pipelined, and/or parallel processing can be utilized as appropriate. In one embodiment, circuitry 42 is provided as one or more components coupled to a dedicated integrated circuit form of DSP 44; processor 46 is provided in the form of one or more general purpose central processing units that interface with DSP 44 over a standard bus connection; and memory 48 includes dedicated memory circuitry integrated within DSP 44 and processor 46, and one or more external memory components including a removable disk form of RMD 48a. Circuitry 42, DSP 44, and/or processor 46 can include one or more signal filters, limiters, oscillators, format converters (such as DACs or ADCs), power supplies, or other signal operators or conditioners as appropriate to operate system 20 in the manner to be described in greater detail hereinafter.

In one embodiment, connection link 50 includes flexible electric cabling 52 with proximal end 52a opposite distal end 52b, and connector 54 electrically connected to equipment 31 of nerve monitoring system 30. Cabling 52 extends from connector 54 at proximal end 52a to distal end 52b where it is connected with device 60. Connection link 50 may include forms in addition to or in alternative to cabling 52, including one or more wires, cords, wireless links, infrared components, bluetooth, or other communication link. Further, it should be appreciated that other components, devices, and systems can be integrated into system 20, such as an endoscope system, a catheterization system, an imaging system, a lighting system, and/or a video camera system, to name a few examples. Connection link 50 and device 60 are movable toward and away from spinal column B in a surgical procedure that may include one or more of retractors, tubes, sleeves, guards, micro-incisions or other components not shown to enhance clarity.

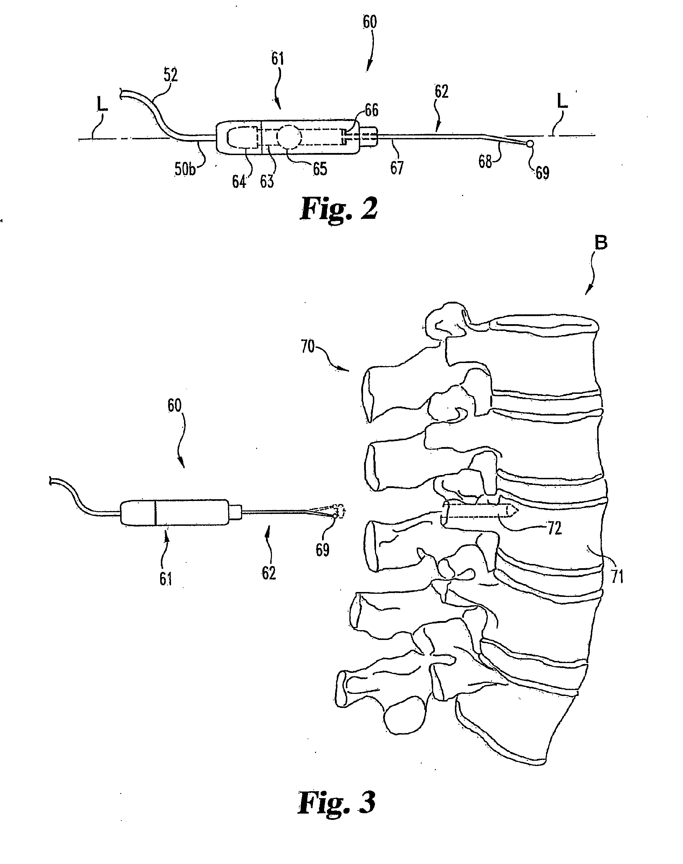

Fig. 2 illustrates one embodiment of device 60. Device 60 extends generally along a longitudinal axis L and includes a handle member 61 and a probe member 62. Handle member 61 is composed of an insulative member or coating surrounding an inner portion 63. In one embodiment, inner portion 63 carries a circuit 64 and a motor 65 which operates to rotate probe member 62 about longitudinal axis L. Inner portion 63 includes a rotatable coupling member 66 at a distal end thereof to operably connect with probe member 62. It should be appreciated that handle member 61 and probe member 62 can be operably, rotatably, and electrically coupled together by any suitable means, including threaded connections, ball-detent type connections, friction fits, slip fits, fasteners, and bayonet locks, for example.

In one embodiment, handle member 61 is operable to indicate to the operator that probe member 62 is coupled thereto. Such connection can switch on a display of the system, a light on the instrument, provide an audible indication, or provide any other suitable indicator. Probe member 62 can be provided in electrical engagement with an electrical current source to determine the proximity of neural elements relative to a location of probe member 62. For example, an electrical lead can extend from probe member 62, through handle member 61, to nerve monitoring system 30.

Probe member 62 includes a longitudinal section 67 at a proximal end and an angled section 68 adjacent a distal end of probe member 62. Angled section 68 includes a non-insulated probe end 69 at a distal end of probe member 62. Probe end 69 can be in the form of a ball tip or dissection or resection member that rotates about longitudinal axis L to sweep the hole formed in the bone tissue. In one embodiment, the ball tip or other probe end is expandable to facilitate in tissue dilation and hole formation in bone tissue. In another embodiment, a set of probe members is provided and attachable with a handle portion. The set of probe members can be provided with ends of various sizes for insertion into the bone tissue for sequential dilation of the hole in the tissue.

In the illustrated embodiment, angled section 68 is generally cylindrically shaped and probe end 69 is generally spherically shaped. Additionally, handle member 61 and longitudinal section 67 of probe member 62 are generally cylindrically shaped about longitudinal axis L. Longitudinal section 67 and angled section 68 are composed of an electrically conductive material with an insulative member or coating thereabout to prevent shunting of electricity delivered therethrough to adjacent tissue or devices. Probe end 69 is not insulated so that the electrical signal carried thereby is exposed to the adjacent bone tissue.

Fig. 3 illustrates the relationship of device 60 to a segment 70 of spinal column B. In the illustrated embodiment, segment 70 includes a vertebra 71 which includes a hole 72 formed in a spinal pedicle. Probe member 62 is configured for insertion in or for forming hole 72 during normal use. After positioning in hole 72, probe end 69 rotates about longitudinal axis L to sweep hole 72 for defects. The proximity of neural elements to probe end 69 in the bone tissue surrounding hole 72 provides an indication of defects that expose neural elements. In an alternative embodiment and alternative operation of system 20, device 60 can also form a hole or bore in the bone tissue while locating defects therein.

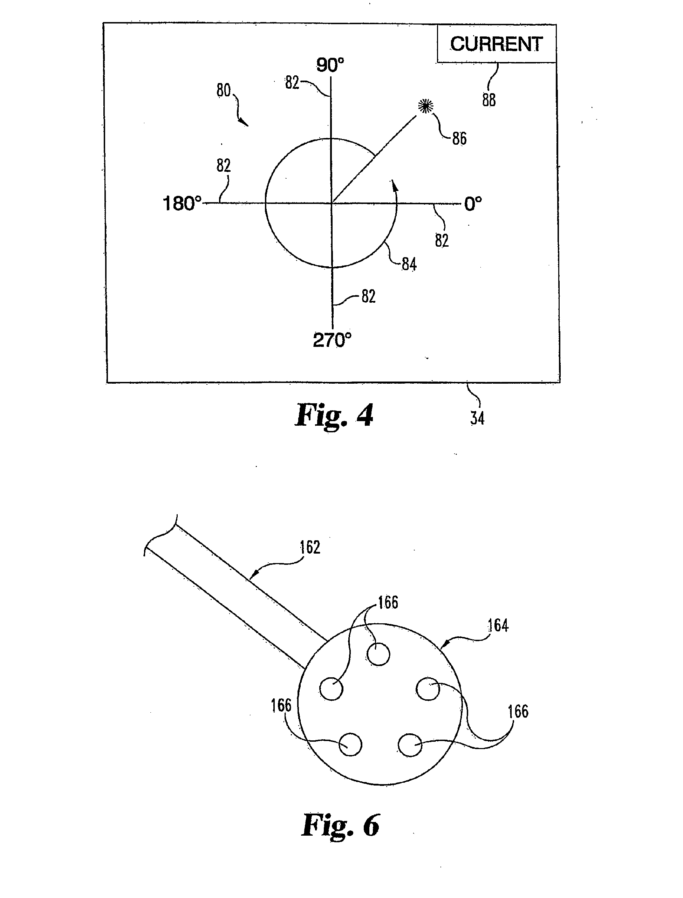

Fig. 4 illustrates one embodiment of a display device 34 during operation of system 20. Display device 34 provides a diagram 80 according to one embodiment. Diagram 80 generally includes four vectors 82 representing positions of 0, 90, 180, and 270 degrees about longitudinal axis L relative to a particular orientation of handle member 61 relative to hole 72. Diagram 80 further includes a representation 84 of the sweeping rotational movement of probe end 69 in hole 72. Diagram 80 may also include an indication of a defect location in the bone tissue around hole 72 with a defect indicator 86. The location of defect indicator 86 relative to vectors 82 enables an operator of system 20 to determine the location of the defect about hole 72 in view of the relative orientation of handle member 61 with hole 72. In one embodiment, defect indicator 86 has a blinking effect on operator display device 34 for increased visibility. Additionally, operator display device 34 may display a representation 88 of the electrical current, voltage or the like supplied to probe end 69.

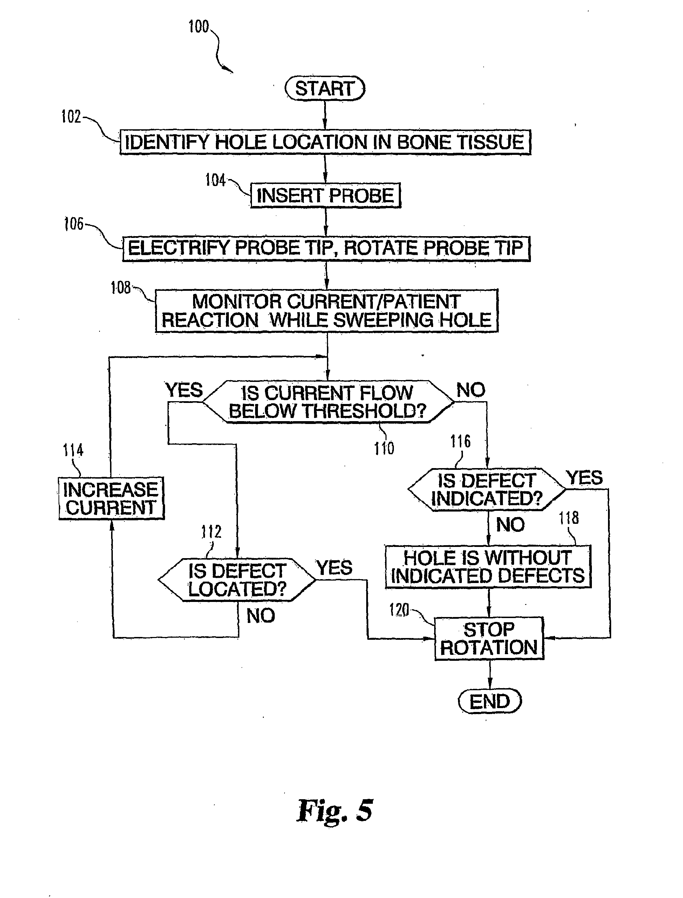

An example of a procedure 100 for operating system 20 is provided in Fig. 5. At stage 102, a hole location for a hole in the bone tissue is identified, such as hole 72 for example. In one embodiment, the bone tissue is an area of tissue within a spinal pedicle wall of vertebra 71 of spinal segment 70. At stage 104, probe end 69 of probe member 62 is inserted into hole 72. The probe end 69 can be manipulated to form an opening in the bone tissue along a particular path. At stage 106, probe end 69 is provided with an electrical signal so that the opening or hole can be swept for identification of defects. Probe end 69 is then rotated by motor 65 about longitudinal axis L. The continuous rotation of probe end 69 is illustrated on operator display device 34 as representation 84. An operator of system 20 can view display device 34 to monitor the rotation of probe end 69 in hole 72. The current at probe end 69 and patient reaction is monitored at stage 108 while probe end 69 continuously rotates about longitudinal axis L within hole 72 to determine the proximity of neural elements. The signal in probe end 69 will invoke a response by the patient that is recorded by system 30 to indicate that a neural element is in close proximity and a defect is present in the bone tissue about the hole. The location of probe end 69 relative to the opening in the bone tissue that results in the response is indicated by defect indicator 86.

Procedure 100 continues at stage 110 where it is determined if the current at probe end 69 is below a predetermined threshold. The predetermined threshold can be the current at probe end 69 that invokes a response from the patient that indicates probe end 69 is sufficiently close in proximity with a neural element that hole 72 should be redirected to avoid the neural element. If the current flow is below the predetermined threshold and there is no indication of the presence of a defect or neural element at stage 112, the current is increased at stage 114 and the procedure is repeated at stage 110.

If at stage 110 the current is determined to not be below the threshold, then the procedure continues at stage 116 where is determined whether a defect or neural element has been indicated. If not, then the procedure continues at stage 118 where the hole is determined to be without an indicated defect, and hole preparation and/or anchor insertion continues. If a defect has been indicated at either of stage 112 or stage 116, a signal can be provided to circuit 64 that stops motor 65 from rotating probe end 69. Accordingly, probe end 69 stops rotating in alignment with the location about the hole in which the defect or neural element has been located, as indicated by the position of defect indicator 86 on operator display device 34. The operator of system 20 can view the relative location of indicator 86 and correlate it with a marker on device 60 to discern the relative location of the defect in hole 72. Another location and hole for insertion of a screw or anchor can be provided, or probe member 62 can be re-directed in a direction away from the location of the defect to re-route or re-direct the hole being formed.

In Fig. 6 there is shown another embodiment probe member 162 which can be attachable to a handle portion, such as handle portion 61. Probe member 162 includes a probe end 164 illustrated in the form of a ball tip but may take any suitable form. Probe member 162 can include an elongated linear shaft portion that may or may not include a distal angled portion as discussed above. Probe end 164 includes a plurality of stimulation elements 166 spaced thereabout. Individual ones of the stimulation elements 166 can be alternately and sequentially energized to spatially deliver an electrical signal to the bone tissue about probe end 164.

The neural stimulation delivered by individual ones of the stimulation elements 166 can be monitored and compared to one another and/or to a known threshold. The current flow or other condition of one or more of the stimulation elements 166 providing neural stimulation can indicate the presence of a defect in the bone wall or tissue that provides an electrical path to the neural element. The identification of the particular stimulation element indicating the presence defect can provide an indication of the location of the defect relative to the longitudinal axis of the probe member. The probe member need not be rotated in the hole in the bone tissue since electrical stimulation is provided and directed from various locations about the probe end.

In another embodiment, one or more stimulation elements create an external electrical field relative to the hole to be probed. The probe end is provided with sensors about its tip that alternately and sequentially measure neural stimulation created by the external stimulation about the hole. The sensor detecting neural stimulation from within the hole provides an indication of the presence and location of a defect in the bone tissue relative to the probe end inserted in the hole in the bone tissue.

While the invention has been illustrated and described in detail in the drawings and foregoing description, the same is to be considered as illustrative and not restrictive in character.

## Claims

1. An instrument (60) for locating defects within bone tissue, comprising:
a handle member (61) including a rotatable coupling member (66); and
a probe member (62) coupled to said coupling member of said handle member and extending distally therefrom, wherein said probe member includes a longitudinal shaft portion (67) along a longitudinal axis (L) and **characterised in that** said probe member includes a distal angled portion (68) extending transversely to said longitudinal axis, wherein said angled portion includes a probe end (69) at a distal end thereof for carrying an electrical signal, wherein said handle member is operable to rotate said coupling member and said probe member and said probe end about said longitudinal axis to locate defects in bone tissue during sweeping rotational movement of the probe end.

2. The instrument of claim 1, wherein said probe end is a ball tip.

3. The instrument of claim 1, wherein said handle member extends along said longitudinal axis.

4. The instrument of claim 1, further comprising an electrical lead extending from said probe member through said handle member.

5. The instrument of claim 1, wherein said longitudinal shaft portion and said distal angled portion are composed of an electrically conductive material and are insulated.

6. The instrument of claim 5, wherein said probe end is non-insulated.

7. The instrument of claim 1, wherein at least said distal angled portion of said probe member is structured for positioning in a hole formed in a spinal pedicle wall to receive an anchor.

8. The instrument of claim 1, wherein said handle member includes a motor operable to rotate said coupling member and said probe member about said longitudinal axis.

9. The instrument of claim 1, wherein said handle member includes a circuit and a motor operable to rotate said probe member about the longitudinal axis within a first hole in the bone tissue.

10. The instrument of claim 9, wherein said handle member is operable to automatically stop rotation of said probe member at the defect location in the bone tissue.

11. The instrument of claim 1, wherein in operation:
said probe end is operable to stop rotation in alignment with a defect in the bone tissue.

12. The instrument of claim 1, further comprising a connection link extending from a proximal end of said handle member to a nerve monitoring system.

13. The instrument of claim 1, wherein said probe member is operable for rotation about said longitudinal axis when said probe end is positioned in a first hole in the bone tissue.

14. The instrument of claim 13, wherein the instrument is operable to automatically stop rotation of said probe member upon detection of the defect.

15. A neural element detection apparatus, comprising:
the instrument (60) of claim 1, wherein said probe end (69) is operable to rotate about the longitudinal axis to detect neural elements in bone tissue about a first hole in a pedicle wall when inserted in the hole; and
an operator display device (34) to display a representation of the rotation of said probe end relative to the longitudinal axis and a location of a defect indicated by detection of neural elements in the bone tissue about said probe end.

16. The apparatus of claim 15, wherein:
upon detection of the neural element a signal is provided to said locating instrument to stop rotation of said probe end adjacent the defect; and
said operator display device is operable to display a location of the probe end and thus the defect relative to the longitudinal axis.

17. The apparatus of claim 15, wherein:
said shaft portion and said angled portion are insulated and are composed of an electrically conductive material; and
said probe end is non-insulated.

18. The apparatus of claim 15, further comprising a nerve monitoring system electrically coupled to said locating instrument.

19. A system, comprising:
the instrument (60) of claim 1, wherein said handle member includes a circuit and a motor operable to rotate said coupling member of said handle member and wherein
said probe member (62) includes a proximal end and a distal end opposite said proximal end, wherein said proximal end of said probe member is coupled to said coupling member of said handle member, wherein said longitudinal shaft portion extends along a longitudinal axis at said proximal end and said distal angled portion extends transversely to said longitudinal axis adjacent said distal end, wherein said probe end (69) is operable to detect neural elements in bone tissue; and
a nerve monitoring system electrically coupled to said probe member operable to indicate a proximity of neural elements to said probe end.

20. The system of claim 19, wherein said nerve monitoring system includes an operator display device operable to display a location of said probe end relative to said longitudinal axis.

21. The system of claim 20, wherein upon detection of a neural element a defect in the bone tissue is located and the system is operable to provide a signal to said circuit to stop said motor and rotation of said probe member with said probe end adjacent the defect.

22. The system of claim 21, wherein said operator display device is operable to display a location of the defect relative to said longitudinal axis.

## Patentansprüche

1. Instrument (60) zum Lokalisieren von Defekten in Knochengewebe, umfassend:
ein Griffelement (61) mit einem drehbaren Kopplungselement (66); und
ein Sondenelement (62), das mit dem Kopplungselement des Griffelements gekoppelt ist und sich von diesem erstreckt, wobei das Sondenelement einen longitudinalen Schaftabschnitt (67) entlang einer Längsachse (L) aufweist, und **dadurch gekennzeichnet, dass** das Sondenelement einen distalen angewinkelten Abschnitt (68) aufweist, der sich transversal zu der Längsachse erstreckt, wobei der angewinkelte Abschnitt an einem distalen Ende ein Sondenende (69) zum Führen eines elektrischen Signals aufweist, wobei das Griffelement so funktionsfähig ist, dass es das Kopplungselement und das Sondenelement um die Längsachse dreht, um während einer abtastenden Drehbewegung des Sondenendes Defekte in Knochengewebe zu lokalisieren.

2. Instrument nach Anspruch 1, wobei das Sondenende eine Kugelspitze ist.

3. Instrument nach Anspruch 1, wobei sich das Griffelement entlang der Längsachse erstreckt.

4. Instrument nach Anspruch 1, ferner umfassend eine elektrische Zuleitung, die sich von dem Sondenelement durch das Griffelement erstreckt.

5. Instrument nach Anspruch 1, wobei der longitudinale Schaftabschnitt und der distale angewinkelte Abschnitt aus einem elektrisch leitfähigen Material bestehen und isoliert sind.

6. Instrument nach Anspruch 5, wobei das Sondenende nicht isoliert ist.

7. Instrument nach Anspruch 1, wobei wenigstens der distale angewinkelte Abschnitt des Sondenelements für eine Positionierung in einem Loch strukturiert ist, das in einer spinalen Pedikelwand ausgebildet ist, um einen Anker aufzunehmen.

8. Instrument nach Anspruch 1, wobei das Griffelement einen Motor aufweist, der so funktionsfähig ist, dass er das Kopplungselement und das Sondenelement um die Längsachse dreht.

9. Instrument nach Anspruch 1, wobei das Griffelement einen Schaltkreis und einen Motor aufweist, die so funktionsfähig sind, dass sie das Sondenelement um die Längsachse in einem ersten Loch in dem Knochengewebe drehen.

10. Instrument nach Anspruch 9, wobei das Griffelement so funktionsfähig ist, dass es die Rotation des Sondenelements an der Position des Defekts in dem Knochengewebe automatisch anhält.

11. Instrument nach Anspruch 1, wobei im Betrieb:
das Sondenelement so funktionsfähig ist, dass es die Rotation in Ausrichtung mit einem Defekt in dem Knochengewebe anhält.

12. Instrument nach Anspruch 1, ferner umfassend einen verbindende Verknüpfung, die sich von einem ein proximalen Ende des Griffelements zu einem Nervenüberwachungssystem erstreckt.

13. Instrument nach Anspruch 1, wobei das Sondenelement für eine Rotation um die Längsachse funktionsfähig ist, wenn das Sondenende in einem ersten Loch in dem Knochengewebe positioniert ist.

14. Instrument nach Anspruch 13, wobei das Instrument so funktionsfähig ist, dass es bei Erkennung des Defekts die Rotation des Sondenelements automatisch anhält.

15. Nervenelementerkennungsvorrichtung, umfassend:
das Instrument (60) aus Anspruch 1, wobei das Sondenende (69) so funktionsfähig ist, dass es sich um die Längsachse dreht, um Nervenelemente in Knochengewebe um ein erstes Loch in einer Pedikelwand zu erkennen, wenn es in das Loch eingeführt ist; und
eine Anzeigevorrichtung (34) für eine Bedienungsperson, zum Anzeigen einer Darstellung der Rotation des Sondenendes im Verhältnis zu der Längsachse sowie einer Position eines durch die Erkennung von Nervenelementen in dem Knochengewebe um das Sondenende angezeigten Defekts.

16. Vorrichtung nach Anspruch 15, wobei:
bei Erkennung des Nervenelements ein Signal an das lokalisierende Instrument bereitgestellt wird, um die Rotation des Sondenendes neben dem Defekt anzuhalten; und
wobei die Anzeigevorrichtung für eine Bedienungsperson so funktionsfähig ist, dass sie eine Position des Sondenendes und somit des Defekts im Verhältnis zu der Längsachse anzeigt.

17. Vorrichtung nach Anspruch 15, wobei:
der Schaftabschnitt und der angewinkelte Abschnitt isoliert sind und aus einem elektrisch leitfähigen Material bestehen; und
das Sondenende nicht isoliert ist.

18. Instrument nach Anspruch 1, wobei diese ferner ein Nervenüberwachungssystem umfasst, das mit dem lokalisierenden Instrument gekoppelt ist.

19. System, umfassend:
das Instrument (60) aus Anspruch 1, wobei das Griffelement einen Schaltkreis und einen Motor aufweist, der so funktionsfähig ist, dass er das Kopplungselement um das Griffelement dreht; und wobei
das Sondenelement (62) ein proximales Ende und ein distales Ende entgegengesetzt zu dem proximalen Ende aufweist, wobei das proximale Ende des Sondenelements mit dem Kopplungselement des Griffelements gekoppelt ist, wobei sich der longitudinale Schaftabschnitt entlang einer Längsachse an dem proximalen Ende erstreckt, und wobei sich der distale angewinkelte Abschnitt transversal zu der Längsachse angrenzend an das distale Ende erstreck, wobei das Sondenende (69) so funktionsfähig ist, dass es Nervenelemente in Knochengewebe erkennt; und
ein Nervenüberwachungssystem, das mit dem Sondenelement gekoppelt und so funktionsfähig ist, dass es eine Proximität von Nervenelementen zu dem Sondenende anzeigt.

20. System nach Anspruch 19, wobei das Nervenüberwachungssystem eine Anzeigevorrichtung für eine Bedienungsperson aufweist, die so funktionsfähig ist, dass sie eine Position des Sondenendes im Verhältnis zu der Längsachse anzeigt.

21. System nach Anspruch 20, wobei bei Erkennung eines Nervenelements ein Defekt in dem Knochengewebe lokalisiert wird, und wobei das System so funktionsfähig ist, dass es ein Signal an den Schaltkreis bereitstellt, um den Motor und die Rotation des Sondenelements mit dem Sondenende neben dem Defekt anzuhalten.

22. System nach Anspruch 21, wobei die Anzeigevorrichtung für einen Benutzer so funktionsfähig ist, dass sie eine Position des Defekts im Verhältnis zu der Längsachse anzeigt.

## Revendications

1. Instrument (60) pour localiser les anomalies dans le tissu osseux, comprenant :
un élément poignée (61) comprenant un élément d'accouplement rotatif (66) ; et
un élément sonde (62) accouplé audit élément d'accouplement dudit élément poignée et s'étendant distalement de celui-ci, dans lequel ledit élément sonde comprend une partie d'arbre longitudinale (67) le long d'un axe longitudinal (L) et **caractérisé en ce que** ledit élément sonde comprend une partie coudée distale (68) s'étendant transversalement vers ledit axe longitudinal, dans lequel ladite partie coudée comprend une extrémité de sonde (69) à son extrémité distale pour porter un signal électrique, dans lequel ledit élément poignée permet de faire tourner ledit élément d'accouplement et ledit élément sonde et ladite extrémité de sonde autour dudit axe longitudinal pour localiser des anomalies dans le tissu osseux pendant le mouvement rotatif de balayage de l'extrémité de sonde.

2. Instrument selon la revendication 1, dans lequel ladite extrémité de la sonde est une extrémité sphérique.

3. Instrument selon la revendication 1, dans lequel ledit élément poignée s'étend le long dudit axe longitudinal.

4. Instrument selon la revendication 1, comprenant en outre un fil électrique s'étendant à partir dudit élément sonde à travers ledit élément poignée.

5. Instrument selon la revendication 1, dans lequel ladite partie d'arbre longitudinale et ladite partie coudée distale sont composées d'un matériau électriquement conducteur et sont isolées.

6. Instrument selon la revendication 5, dans lequel ladite extrémité de sonde est non isolée.

7. Instrument selon la revendication 1, dans lequel au moins ladite partie coudée distale dudit élément sonde est structurée pour un positionnement dans un trou formé dans une paroi de pédicule vertébral pour recevoir un ancrage.

8. Instrument selon la revendication 1, dans lequel ledit élément poignée comprend un moteur permettant de faire tourner ledit élément d'accouplement et ledit élément sonde autour dudit axe longitudinal.

9. Instrument selon la revendication 1, dans lequel ledit élément poignée comprend un circuit et un moteur permettant de faire tourner ledit élément sonde autour de l'axe longitudinal à l'intérieur d'un premier trou dans le tissu osseux.

10. Instrument selon la revendication 9, dans lequel ledit élément poignée permet d'arrêter automatiquement la rotation dudit élément sonde au niveau de la localisation de l'anomalie dans le tissu osseux.

11. Instrument selon la revendication 1, dans lequel, en fonctionnement :
ladite extrémité de sonde permet d'arrêter la rotation en alignement avec une anomalie dans le tissu osseux.

12. Instrument selon la revendication 1, comprenant en outre une liaison de connexion s'étendant d'une extrémité proximale dudit élément poignée à un système de surveillance des nerfs.

13. Instrument selon la revendication 1, dans lequel ledit élément sonde peut tourner autour dudit axe longitudinal lorsque ladite extrémité de sonde est positionnée dans un premier trou dans le tissu osseux.

14. Instrument selon la revendication 13, dans lequel l'instrument permet d'arrêter automatiquement la rotation dudit élément sonde lors de la détection de l'anomalie.

15. Appareil de détection d'élément neuronal, comprenant :
l'instrument (60) selon la revendication 1, dans lequel ladite extrémité de sonde (69) peut tourner autour de l'axe longitudinal pour détecter des éléments neuronaux dans le tissu osseux autour d'un premier trou dans une paroi de pédicule lorsqu'elle est insérée dans le trou ; et
un dispositif d'affichage d'opérateur (34) pour afficher une représentation de la rotation de ladite extrémité de sonde par rapport à l'axe longitudinal et une localisation d'une anomalie indiquée par la détection d'éléments neuronaux dans le tissu osseux autour de ladite extrémité de sonde.

16. Appareil selon la revendication 15, dans lequel :
lors de la détection de l'élément neuronal, un signal est fourni audit instrument de localisation pour arrêter la rotation de ladite extrémité de sonde à côté de l'anomalie ; et
ledit dispositif d'affichage d'opérateur permet d'afficher une localisation de l'extrémité de sonde et ainsi de l'anomalie par rapport à l'axe longitudinal.

17. Appareil selon la revendication 15, dans lequel :
ladite partie d'arbre et ladite partie coudée sont isolées et comprennent un matériau électriquement conducteur ; et
ladite extrémité de sonde est non isolée.

18. Appareil selon la revendication 15, comprenant en outre un système de surveillance de nerfs couplé électriquement audit instrument de localisation.

19. Système, comprenant :
l'instrument (60) selon la revendication 1, dans lequel ledit élément poignée comprend un circuit et un moteur permettant de faire tourner ledit élément d'accouplement dudit élément poignée et dans lequel
ledit élément sonde (62) comprend une extrémité proximale et une extrémité distale opposée à ladite extrémité proximale, dans lequel ladite extrémité proximale dudit élément sonde est accouplée audit élément d'accouplement dudit élément poignée, dans lequel ladite partie d'arbre longitudinale s'étend le long d'un axe longitudinal au niveau de ladite extrémité proximale et ladite partie coudée distale s'étend transversalement audit axe longitudinal à proximité de à ladite extrémité distale, dans lequel ladite extrémité de sonde (69) permet de détecter des éléments neuronaux dans le tissu osseux ; et
un système de surveillance des nerfs couplé électriquement audit élément sonde permettant d'indiquer la proximité d'éléments neuronaux par rapport à ladite extrémité de sonde.

20. Système selon la revendication 19, dans lequel ledit système de surveillance de nerfs comprend un dispositif d'affichage d'opérateur permettant d'afficher une localisation de ladite extrémité de sonde par rapport audit axe longitudinal.

21. Système selon la revendication 20, dans lequel, lors de la détection d'un élément neural, une anomalie dans le tissu osseux est localisée et le système permet de fournir un signal audit circuit pour arrêter ledit moteur et la rotation dudit élément sonde avec ladite extrémité de sonde à proximité de l'anomalie.

22. Système selon la revendication 21, dans lequel ledit dispositif d'affichage d'opérateur permet d'afficher une localisation de l'anomalie par rapport audit axe longitudinal.
